(19) Europäisches Patentamt
European Patent Office
Office européen des brevets

(11) Veröffentlichungsnummer: **0 471 646 B1**

# (12) EUROPÄISCHE PATENTSCHRIFT

(45) Veröffentlichungstag der Patentschrift: **13.09.95**

(21) Anmeldenummer: **91810615.4**

(22) Anmeldetag: **06.08.91**

(51) Int. Cl.$^6$: **C07D 251/16**, A01N 47/36, C07D 251/46, C07D 251/42, C07D 239/47, C07D 239/52

## (54) Sulfonylharnstoffe als Herbizide.

(30) Priorität: **15.08.90 CH 2654/90**

(43) Veröffentlichungstag der Anmeldung:
**19.02.92 Patentblatt 92/08**

(45) Bekanntmachung des Hinweises auf die Patenterteilung:
**13.09.95 Patentblatt 95/37**

(84) Benannte Vertragsstaaten:
**CH DE FR GB LI**

(56) Entgegenhaltungen:
**EP-A- 0 044 808**
**EP-A- 0 082 108**

(73) Patentinhaber: **CIBA-GEIGY AG**
**Klybeckstrasse 141**
**CH-4002 Basel (CH)**

(72) Erfinder: **Meyer, Willy**
**Talweg 49**
**CH-4125 Riehen (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 471 646 B1

## Beschreibung

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Phenylsulfonylharnstoffe mit herbizider Wirkung sind aus der Europäischen Patentanmeldung Nr. 0 044 808 bekannt. Die dort konkret offenbarten Wirkstoffe können jedoch die Anforderungen bezüglich Wirkungsstärke und Wirkungsspektrum nicht immer erfüllen. Es besteht somit ein Bedarf nach besser wirksamen und selektiveren Wirkstoffen.

Es wurden nun neue Sulfonylharnstoffe mit verbesserten herbiziden und pflanzenwachstumsregulierenden Eigenschaften gefunden.

Die erfindungsgemäßen N-Phenylsulfonyl-N'-pyrimidinyl- und -triazinylharnstoffe entsprechen der Formel I

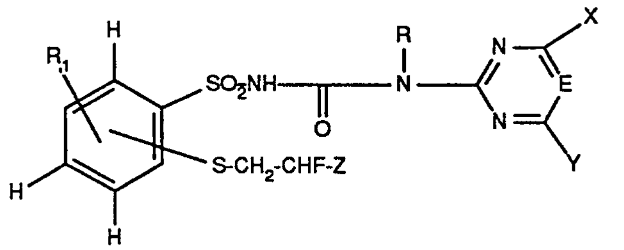

(I)

worin

R Wasserstoff oder $C_1$-$C_4$-Alkyl;

$R_1$ Wasserstoff, Halogen, $C_1$-$C_5$-Alkyl, $C_2$-$C_5$-Alkenyl, $C_1$-$C_4$-Halogenalkyl, COO-$R_2$ oder A-$R_3$;

$R_2$ $C_1$-$C_5$-Alkyl;

$R_3$ $C_1$-$C_5$-Alkyl, $C_2$-$C_4$-Alkoxyalkyl oder $C_1$-$C_5$-Halogenalkyl;

X $C_1$-$C_3$-Alkyl, durch Halogen ein- bis dreifach substituiertes $C_1$-$C_3$-Alkyl; $C_1$-$C_3$-Alkoxy oder durch Halogen ein- bis dreifach substituiertes $C_1$-$C_3$-Alkoxy;

A Sauerstoff, Schwefel, SO oder $SO_2$;

Y Halogen, $C_1$-$C_3$-Alkyl, durch Halogen ein- bis dreifach substituiertes $C_1$-$C_3$-Alkyl; $C_1$-$C_3$-Alkoxy, durch Halogen ein- bis dreifach substituiertes $C_1$-$C_3$-Alkoxy; Cyclopropyl, Methylamino oder Dimethylamino;

E Stickstoff oder die Methingruppe; und

Z Fluor oder Chlor bedeuten;

sowie die Salze dieser Verbindungen.

Die in den Substituentendefinitionen vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, n-Pentyl oder die isomeren Pentyl. Vorzugsweise steht Alkyl im Rahmen der vorliegenden Erfindung für Methyl oder Ethyl. Unter Halogen ist Fluor, Chlor, Brom und Jod, vorzugsweise Fluor und Chlor zu verstehen.

Halogenalkyl steht beispielsweise für Trifluormethyl, 1-Fluorethyl, 1,1-Dichlorethyl, 3,3,3-Trifluorpropyl, 2-Fluorisopropyl, 3-Fluorpropyl, 1,1,1-Trichlorpentyl, 1-Fluor-3-methylpentyl, oder 1-Bromhexyl.

Beispiele für Alkoxyalkyl sind: Methoxymethyl, Methoxyethyl, Methoxypropyl, Ethoxymethyl, Ethoxyethyl oder Propyloxymethyl.

Die als oder in den Substituenten X und Y vorkommenden $C_1$-$C_3$-Alkylreste umfassen im einzelnen Methyl, Ethyl, n-Propyl und iso-Propyl sowie die von diesen Resten abgeleiteten ein- bis dreifach durch Halogen substituierten Halogenalkyle. Vorzugsweise besitzen die als oder in den Substituenten X und Y vorkommenden Alkylreste ein bis zwei Kohlenstoffatome.

Bei den als oder in den Substituenten X und Y vorkommenden ein- bis dreifach durch Halogen substituierten $C_1$-$C_3$-Alkylgruppen sind ein- bis dreifach durch Fluor oder Chlor substituierte $C_1$-$C_2$-Alkylgruppen bevorzugt. Speziell bevorzugte, als oder in den Substituenten X und Y vorkommende, ein- bis dreifach durch Halogen substituierte $C_1$-$C_3$-Alkylreste sind: Trifluormethyl, Difluormethyl, 2-Chlorethyl, Chlordifluormethyl,

Dichlormethyl, Chlorfluormethyl, 1,1-Dichlorethyl, Trifluorethyl, 3,3,3-Trifluorpropyl oder 2,3-Dichlorpropyl, besonders bevorzugt sind Fluormethyl, Chlormethyl, Difluormethyl und Trifluormethyl.

Die im Substituenten $R_1$ vorkommenden $C_2$-$C_5$-Alkenylreste können in der Z-Form (cis) oder in der E-Form (trans) vorliegen und können geradkettig oder verzweigt sein. Bevorzugt sind Alkenylreste mit einer Kettenlänge von zwei bis drei Kohlenstoffatomen. Beispiele für $C_2$-$C_5$-Alkenylreste sind: Vinyl, Allyl, Methallyl, 1-Methylvinyl, 2-Methylvinyl, But-2-en-1-yl oder Pent-3-en-1-yl. Bevorzugt ist Vinyl und Allyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, iso-Propylamin, die vier isomeren Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und iso-Chinolin, insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Unter den Verbindungen der Formel I sind diejenigen bevorzugt, in denen $R_1$ am Phenylring in 5-Stellung und die Gruppe -S-$CH_2$-CHF-Z in 2-Stellung steht.

Ferner sind unter den Verbindungen der Formel I diejenigen bevorzugt, in denen $R_1$ und/oder R Wasserstoff bedeutet.

Ganz besonders bevorzugte Gruppen von Verbindungen der Formel I sind jene, in welchen

a) X für $C_1$-$C_3$-Alkyl, $C_1$-$C_3$-Alkoxy oder durch Halogen ein- bis dreifach substituiertes $C_1$-$C_3$-Alkoxy; und Y für Chlor, $C_1$-$C_3$-Alkyl, $C_1$-$C_3$-Alkoxy, Cyclopropyl oder durch Halogen ein- bis dreifach substituiertes $C_1$-$C_3$-Alkoxy steht; oder

b) X für Methyl, Methoxy, Ethoxy oder Difluormethoxy; und Y für Methyl, Methoxy, Ethoxy, Difluormethoxy oder Chlor steht; oder

c) X für Methoxy oder Ethoxy; und Y für Methyl oder Methoxy steht; oder

d) E Stickstoff bedeutet.

Eine durch ihre gute biologische Wirksamkeit besonders herausragende Gruppe von Verbindungen der Formel I ist jene, bei welcher $R_1$ für Wasserstoff steht und X für $C_1$-$C_3$-Alkyl, $C_1$-$C_3$-Alkoxy oder durch Halogen ein- bis dreifach substituiertes $C_1$-$C_3$-Alkoxy; und Y für Chlor, $C_1$-$C_3$-Alkyl, $C_1$-$C_3$-Alkoxy, Cyclopropyl oder durch Halogen ein- bis dreifach substituiertes $C_1$-$C_3$-Alkoxy steht. Aus dieser Gruppe sind ferner diejenigen Verbindungen der Formel I bevorzugt, in denen X für Methyl, Methoxy, Ethoxy oder Difluormethoxy und

Y für Methyl, Methoxy, Ethoxy, Difluormethoxy oder Chlor steht; wobei für X die Bedeutungen Methoxy oder Ethoxy und für Y die Bedeutungen Methyl oder Methoxy ganz besonders bevorzugt sind. Vorzugsweise steht bei dieser herausragenden Gruppe von Verbindungen $R_1$ am Phenylring in 5-Stellung und die Gruppe -S-$CH_2$-CHF-Z in 2-Stellung.

Als bevorzugte Einzelverbindung aus dem Umfang der Formel I sind zu nennen: N-(2-(2-chlor-2-fluorethylthio)-phenyl-sulfonyl)-N'-(4-methoxy-6-methyl-1,3,5-triazin-2-yl)-harnstoff; und N-(2-(2,2-difluor-ethylthio)-phenyl-sulfonyl)-N'-(4-methoxy-6-methyl-1,3,5-triazin-2-yl)-harnstoff.

Die Verbindungen der Formel I können hergestellt werden, indem man

a) ein Phenylsulfonamid der Formel XIV

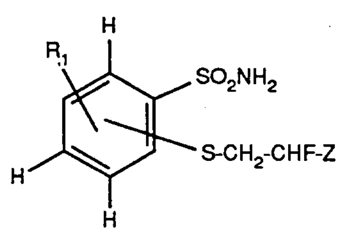

(XIV)

worin $R_1$ und Z die unter Formel I angegebene Bedeutung hat,

mit einem N-Pyrimidinyl- oder N-Triazinylcarbamat der Formel XI

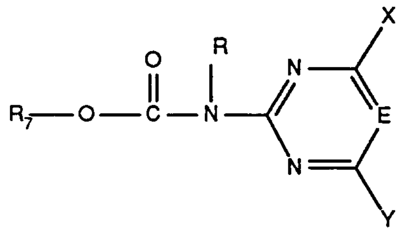

(XI)

worin X, Y, R und E die unter Formel I angegebene Bedeutung haben und $R_7$ $C_1$-$C_4$-Alkyl, oder Phenyl, welches durch $C_1$-$C_4$-Alkyl oder Halogen substituiert sein kann, bedeutet; in Gegenwart einer Base umsetzt, oder

b) ein Phenylsulfonamid der Formel XII

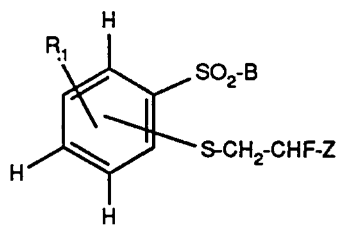

(XII)

worin $R_1$ und Z die in Formel I angegebene Bedeutung haben, und B

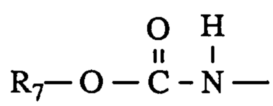

oder O=C=N- bedeutet, in Gegenwart einer Base mit einem 2-Aminopyrimidin- oder -triazin der Formel XIII

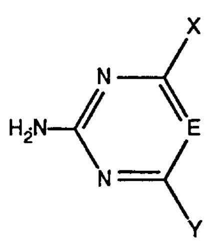

(XIII)

worin E, X und Y die unter Formel I angegebene Bedeutung haben, umsetzt.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie Dimethylformamid, Diethylformamid oder N-Methylpyrrolidinon. Die Reaktionstemperaturen liegen vorzugsweise zwischen -20° und +120°C.

Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten

können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die Zwischenprodukte der Formel XI, XII, und XIII sind bekannt oder können analog zu bekannten Verfahren hergestellt werden. Verfahren zur Herstellung von N-Pyrimidinyl- und N-Triazinylcarbamaten sind beispielsweise in EP-A-0101670 beschrieben. Verfahren zur Herstellung der Verbindungen der Formel XII sind in EP-A 0 044 808 beschrieben. Verbindungen der Formel XIII sind aus EP-A-0 070 804 bekannt.

Die Verbindungen der Formel XIV können hergestellt werden, indem man

a) ein 2-Halogenphenylsulfonamid der Formel II

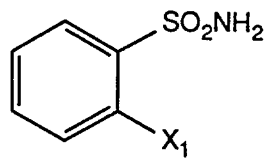

(II)

worin $X_1$ für Fluor, Chlor oder Brom steht, in Gegenwart einer Base mit einem Mercaptan der Formel III

$R_5$ - SH (III)

worin $R_5$ für $C_1$-$C_6$-Alkyl oder durch Phenyl substituiertes $C_1$-$C_6$-Alkyl steht, zu einem 2-Sulfenylphenylsulfonamid der Formel IV

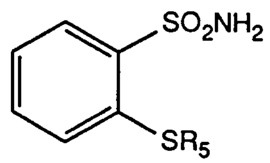

(IV) .

worin $R_5$ die unter Formel III angegebene Bedeutung hat, überführt,

b) dieses zum 2-Sulfinyl-phenylsulfonamid der Formel V

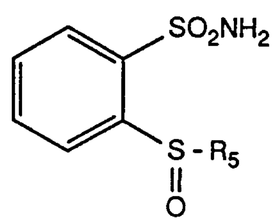

(V)

worin $R_5$ die unter Formel III angegebene Bedeutung hat, oxidiert,

c) das erhaltene 2-Sulfinyl-phenylsulfonamid der Formel V in Gegenwart einer Säure zum Disulfid der Formel VI

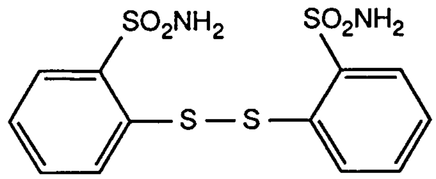

(VI)

umsetzt,

d) das Disulfid der Formel VI zum 2-Mercapto-phenylsulfonamid der Formel VII

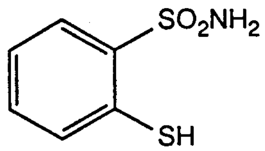

(VII)

reduziert,

e) dieses anschliessend mit einem Trialkylamin der Formel X

$(R_6)_3N$ (X)

worin $R_6$ für $C_1$-$C_4$-Alkyl steht, in das 2-Mercapto-phenylsulfonamid-Trialkylaminsalz der Formel VIII

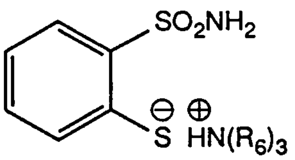

(VIII)

worin $R_6$ die unter Formel X genannte Bedeutung hat, überführt und

f) dieses anschliessend mit einem Halogen-fluorethan der Formel IX

$Y_1$-$CH_2CHF$-Z (IX)

worin $Y_1$ für Chlor oder Brom steht und Z Fluor oder Chlor bedeutet, umsetzt.

Die Verbindungen der Formeln II, III, IV, V, VII und IX sind bekannt und teilweise im Handel erhältlich.

Die Disulfide der Formel VI sowie die 2-Mercapto-phenylsulfonamid-Trialkylaminsalze der Formel VIII sind Gegenstand der schweizerischen Patentanmeldung Nr.3554/89-5.

Die im Substituenten $R_5$ vorkommenden $C_1$-$C_6$-Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, n-Pentyl oder die isomeren Pentyl, n-Hexyl oder die isomeren Hexyl. Vorzugsweise besitzen die im Substituenten $R_5$ vorkommenden Alkylgruppen 1 bis 3 Kohlenstoffatome. Sind die Alkylgruppen durch Phenyl substituiert, besitzen sie vorzugsweise eine Kettenlänge von 1 bis 3 Kohlenstoffatomen. Besonders bevorzugt steht der Substituent $R_5$ für Benzyl.

Die in den Substituenten $R_6$ vorkommenden $C_1$-$C_4$-Alkylgruppen können geradkettig oder verzweigt sein und stehen für Methyl, Ethyl, n-Propyl, iso-Propyl, n-Butyl, sek.-Butyl oder tert.-Butyl. Besonders bevorzugt steht $R_6$ jeweils für Ethyl.

Der Verfahrensschritt a) wird mit Vorteil in einem inerten Lösungsmittel bei einer Temperatur zwischen +20 °C und der Siedetemperatur des Lösungsmittels durchgeführt. Üblicherweise liegen die Temperaturen zwischen +20 und +180 °C, vorzugsweise zwischen +20 und +120 °C. Ein besonders bevorzugter Temperaturbereich liegt zwischen +50 und +70 °C.

Geeignete Lösungsmittel sind chlorierte Kohlenwasserstoffe, wie Dichlormethan, Trichlormethan, Trichlorethan oder Tetrachlorethan, Chlorbenzol oder Dichlorbenzol; aromatische Kohlenwasserstoffe wie Benzol, Toluol oder Xylol; Ether wie Diethylether, Diisopropylether, Tetrahydrofuran oder Dioxan; Nitrile wie Acetonitril oder Propionitril; Cyclohexan, Pyridin, N-Methylpyrrolidon oder N,N-Dimethylformamid wobei N,N-Dimethylformamid besonders bevorzugt ist.

Als Basen werden insbesondere Hydride, Hydroxide, Carbonate oder Alkoholate eines Alkali oder Erdalkalimetalls, ein Trialkylamin oder eine Pyridinbase verwendet. Besonders bevorzugte Basen sind Pyridin, Natriumhydroxid, Natriummethylat, Natriumethylat, Natriumcarbonat oder Kaliumcarbonat. Ganz besonders bevorzugt ist Kaliumcarbonat.

Der Verfahrensschritt a) kann in besonders vorteilhafter Weise durchgeführt werden, wenn man als 2-Halogenphenylsulfonamid der Formel II das 2-Fluor-phenylsulfonamid einsetzt.

Als Oxidationsmittel für den Verfahrensschritt b) sind insbesondere Permanganate, Perjodate, Persäuren oder Wasserstoffperoxid geeignet

Bevorzugte Oxidationsmittel sind Peressigsäure, Perbenzoesäure, Perjodsäure, Kaliumpermanganat, Kaliumperjodat und Wasserstoffperoxid. Ein ganz besonders bevorzugtes Oxidationsmittel ist Wasserstoffperoxid. Die Oxidation des 2-Sulfenylphenylsulfonamids der Formel IV wird mit Vorteil bei Temperaturen von 0° bis +80°C durchgeführt, wobei ein Temperaturbereich von 0° bis +40°C bevorzugt wird.

In einer bevorzugten Variante des Verfahrens zur Herstellung der Verbindungen der Formel II wird die Oxidation des 2-Sulfenylphenylsulfonamids der Formel IV mit Wasserstoffperoxid in Gegenwart von Essigsäure bei einer Temperatur von +5 bis +15°C durchgeführt.

Der Verfahrensschritt c) wird vorzugsweise bei einer Temperatur von +20°C bis zur Siedetemperatur des Lösungsmittels durchgeführt. Als besonders gut geeignete Lösungsmittel haben sich Alkohole mit einer Kettenlänge von 1 bis 4 Kohlenstoffatomen erwiesen, wie zum Beispiel Methanol, Ethanol, Propanol, iso-Propanol, Butanol oder 2-Butanol. Ein bevorzugtes Lösungsmittel ist Methanol.

Säurekatalysierte Umlagerungen von Sulfoxiden sind in der Literatur unter dem Namen "Pummerer Umlagerungen" bekannt. Beispiele für derartige Reaktionen finden sich in Adv. Org. Chem. 6, 356(1969). Im Gegensatz zu der aus diesem Artikel bekannten Lehre führt die säurekatalysierte Umlagerung des 2-Sulfinyl-phenylsulfonamids der Formel V nicht zum 2-Mercapto-phenylsulfonamid sondern zum 2,2'-Bis-aminosulfonyl-diphenyl-disulfid der Formel VI, welches aus seinem Reaktionsmedium auf einfache Weise isoliert werden kann und eine hohe Lagerstabilität aufweist.

Die Reaktion ist bezüglich der Natur der als Katalysatoren verwendeten Säuren unkritisch. Bevorzugte Säuren sind Mineralsäuren, insbesondere Salzsäure oder Schwefelsäure.

Die Verfahrensschritte b) und c) können auch unmittelbar nacheinander ohne Isolierung des Zwischenproduktes der Formel V in einem Reaktionsgefäß durchgeführt werden. Die für diese Verfahrensvariante geeigneten Lösungsmittel entsprechen den in Stufe c) genannten.

Die reduktive Spaltung des Disulfids der Formel VI zum 2-Mercapto-phenylsulfonamid der Formel VII (Verfahrensschritt d)) erfolgt im allgemeinen bei Temperaturen von +20°C bis +100°C.

Die Reduktion wird vorzugsweise mit Wasserstoff in Gegenwart von Edelmetallkatalysatoren, oder mit Zink, Eisen oder Zinn in Gegenwart von Salzsäure oder Essigsäure, oder mit Natrium-, Magnesium- oder Aluminiumamalgam durchgeführt. Bevorzugte Reduktionsmittel sind Wasserstoff in Gegenwart von Platin-, Palladium-, Rhodium- oder Nickelkatalysatoren sowie Zink, Eisen und Zinn in Gegenwart von Salzsäure oder Essigsäure. Ein ganz besonders bevorzugtes Reduktionsmittel ist Zink in Gegenwart von Salzsäure oder Essigsäure.

Ein für die Salzbildung gemaß Verfahrensschritt e) besonders gut geeignetes Trialkylamin der Formel X ist das Triethylamin.

Die Reaktionstemperaturen bei der Umsetzung der 2-Mercapto-phenylsulfonamid-Trialkylaminsalze mit dem Halogen-fluorethan der Formel IX (Verfahrensschritt f)) liegen zwischen 0° und +80°C, bevorzugt zwischen 0° und +40°C. Die Reaktion verläuft besonders vorteilhaft, wenn in der Formel IX $Y_1$ für Brom steht. Die für die Stufe f) geeigneten Lösungsmittel entsprechen den in Stufe a) genannten.

Die Wirkstoffe der Formel I werden in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemäßen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Straßenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodaß die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.

b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln,

wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzähl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen. Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addükten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, die Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyethylenglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die herbiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

| Emulgierbare Konzentrate: | |
|---|---|
| Aktiver Wirkstoff:<br>oberflächenaktives Mittel:<br>flüssiges Trägermittel: | 1 bis 90 %, vorzugsweise 5 bis 20 %<br>1 bis 30 %, vorzugsweise 10 bis 20 %<br>5 bis 94 %, vorzugsweise 70 bis 85 % |

| Stäube: | |
|---|---|
| Aktiver Wirkstoff:<br>festes Trägermittel: | 0,1 bis 10 %, vorzugsweise 0,1 bis 1 %<br>99,9 bis 90 %, vorzugsweise 99,9 bis 99 % |

| Suspensions-Konzentrate: | |
|---|---|
| Aktiver Wirkstoff:<br>Wasser:<br>oberflächenaktives Mittel: | 5 bis 75 %, vorzugsweise 10 bis 50 %<br>94 bis 24 %, vorzugsweise 88 bis 30 %<br>1 bis 40 %, vorzugsweise 2 bis 30 % |

| Benetzbare Pulver: | |
|---|---|
| Aktiver Wirkstoff:<br>oberflächenaktives Mittel:<br>festes Trägermaterial: | 0,5 bis 90 %, vorzugsweise 1 bis 80 %<br>0,5 bis 20 %, vorzugsweise 1 bis 15 %<br>5 bis 95 %, vorzugsweise 15 bis 90 % |

| Granulate: | |
|---|---|
| Aktiver Wirkstoff:<br>festes Trägermittel: | 0,5 bis 30 %, vorzugsweise 3 bis 15 %<br>99,5 bis 70 %, vorzugsweise 97 bis 85 % |

Herstellungsbeispiele:

Beispiel H1: Herstellung von 2-Benzylthio-phenylsulfonamid:

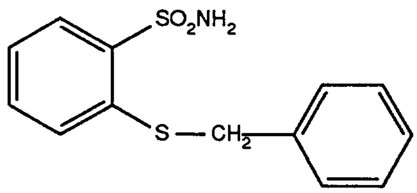

Zu einer Lösung von 124,2 g Benzylmercaptan in 400 ml N,N-Dimethylformamid gibt man 175,2 g 2-Fluorphenylsulfonamid und 160 g Kaliumcarbonat. Anschließend wird die Reaktionsmischung für 3 Stunden auf +60°C erwärmt. Nach Abkühlen der Mischung auf +25°C wird filtriert und das Filtrat anschließend eingedampft.

Der so erhaltene Rückstand wird mit 1500 ml Wasser versetzt, wobei das Produkt in Form farbloser Kristalle ausfällt. Nach Abtrennen der Lösung werden die erhaltenen Kristalle in Eßigsäureethylester gelöst und anschließend mit Magnesiumsulfat behandelt. Nach Abfiltrieren und Eindampfen des Filtrates erhält man 219 g (78,5 % d. Th.) 2-Benzylthio-phenylsulfonamid in Form farbloser Kristalle mit einem Schmelzpunkt von +104 bis +106°C.

Beispiel H2: Herstellung von 2-Benzylsulfinyl-phenylsulfonamid:

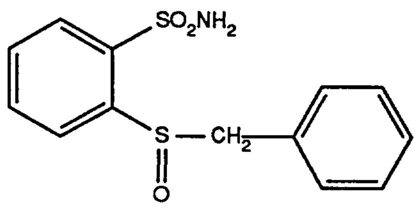

Zu einer Lösung von 219 g gemäß Beispiel H1 erhaltenen 2-Benzylthiophenylsulfonamids in 500 ml konzentrierter Essigsäure gibt man bei einer Temperatur von +10°C 102 ml 30%ige Wasserstoffperoxidlösung tropfenweise hinzu. Anschließend wird die Reaktionsmischung bei einer Temperatur von +25°C 5 Stunden lang gerührt wobei das Produkt langsam auskristallisiert. Nach Abtrennen der Kristalle, Waschen mit Wasser und Trocknen erhält man 219 g (94,6 %) 2-Benzylsulfinyl-phenylsulfonamid in Form farbloser Kristalle mit einem Schmelzpunkt von +206 bis +209°C.

Beispiel H3: Herstellung von 2,2'-Bis-aminosulfonyl-diphenyl-disulfid:

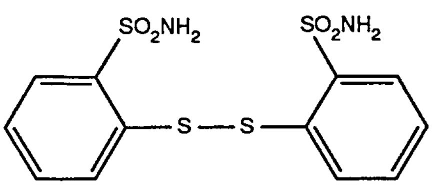

(VI).

Zu einer Lösung aus 214 g eines gemäß Beispiel H2 hergestellten 2-Benzylsulfinyl-phenylsulfonamids in 500 ml Methanol gibt man 500 ml konzentrierte Salzsäure hinzu. Nach 7-stündigem Sieden der Reaktionsmischung und 2-tägigem Halten der Mischung bei Raumtemperatur wird das ausgefallene Disulfid abgetrennt und mit Wasser, Isopropanol und Diethylether gewaschen. Man erhält 133,2 g (97,7 % d. Th.) 2,2'-Bis-aminosulfonyl-diphenyl-disulfid in Form gelblicher Kristalle mit einem Schmelzpunkt von +217 °C (Zers.).

Beispiel H4: Herstellung von 2,2'-Bis-aminosulfonyl-diphenyl-disulfid:

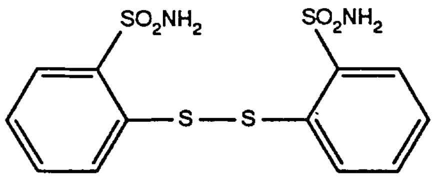

(VI).

Zu einer Lösung von 10 g gemäß Beispiel H1 erhaltenen 2-Benzylthiophenylsulfonamids in 50 ml Ethanol gibt man tropfenweise 50 ml konzentrierte Salzsäure und 3,5 g 30%ige Wasserstoffperoxidlösung hinzu. Anschließend wird die Reaktionsmischung für die Dauer von 1 Stunde auf Rückflußtemperatur erwärmt. Nach Abkühlen auf +25 °C werden die entstandenen Kristalle abgetrennt und mit Isopropanol und Petrolether gewaschen. Man erhält 4 g 2,2'-Bis-aminosulfonyl-diphenyldisulfid in Form gelber Kristalle mit einem Schmelpunkt von +211 bis +213 °C.

Beispiel H5: Herstellung von 2-Mercapto-phenylsulfonamid:

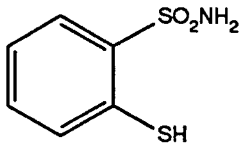

(VII)

Eine Suspension aus 23,2 g eines gemäß Beispiel H3 und H4 erhaltenen 2,2'-Bis-aminosulfonyl-diphenyl-disulfids in 180 ml konzentrierter Essigsäure versetzt man mit 18 g Zinkpulver und erhitzt 30 Minuten auf Rückflußtemperatur. Anschließend wird die Suspension auf +17 °C gekühlt und filtriert. Nach Waschen des Filterrückstandes mit Essigsäureethylester wird das Filtrat eingedampft und der Rückstand anschließend in 200 ml Essigsäureethylester gelöst. Nach zweimaligem Waschen mit 100 ml Wasser und Trocknen mit Magnesiumsulfat wird die Lösung eingedampft. Man erhält 22 g (94% d. Th.) ungereinigtes 2-Mercapto-phenylsulfonamid mit einem Schmelzpunkt von +113 bis +140 °C.

Beispiel H6: Herstellung von 2-Mercapto-phenylsulfonamid-Triethylaminsalz:

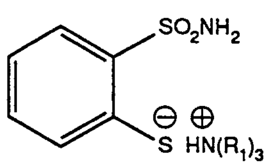

(VIII).

Zu einer Lösung von 7,6 g gemäß Beispiel H5 erhaltenen 2-Mercaptosulfonamids in 60 ml Tetrahydrofuran gibt man bei einer Temperatur von +25 °C tropfenweise 5 g Triethylamin hinzu. Anschließend werden die ausgefallenen, farblosen Kristalle abgetrennt und mit Diethylether gewaschen. Man erhält 9,2 g 2-Mercapto-phenylsulfonamid-Triethylaminsalz mit einem Schmelzpunkt von +164 bis +168 °C.

Beispiel H7: Herstellung von 2-(2,2-Difluorethylthio)-phenyl-sulfonamid:

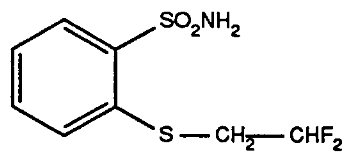

Eine Mischung aus 145,2 g des gemäß Beispiel H6 erhaltenen 2-Mercapto-phenylsulfonamid-Triethylaminsalzes und 79,7 g 2-Brom-1,1-difluorethan in 600 ml Methanol läßt man bei einer Temperatur von +55 °C bis +60 °C 16 Stunden lang rühren. Anschließend wird die Reaktionsmischung eingedampft. Durch Verrühren des Rückstandes mit Eiswasser und Filtrieren der so erhaltenen Suspension erhält man 120 g (95 % d. Th.) 2-(2,2-Difluorethylthio)-phenyl-sulfonamid mit einem Schmelzpunkt von +111 °C bis +112 °C.

Beispiel H8: Herstellung von 2-(2-Chlor-2-fluorethylthio)-phenyl-sulfonamid:

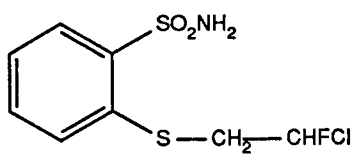

Eine Mischung aus 217,8 g des gemäß Beispiel H6 erhaltenen 2-Mercapto-phenylsulfonamid-Triethylaminsalzes und 101 g 1,2-Dichlor-1-fluorethan in 950 ml Methanol läßt man bei einer Temperatur von +65 °C bis +70 °C 24 Stunden und bei +95 °C bis +100 °C weitere 24 Stunden in einem Autoklaven rühren. Anschließend wird die Reaktionsmischung eingedampft und der Rückstand mit Wasser verrührt. Durch Extrahieren mit Essigsäureethylester, Waschen mit Wasser, Trocknen über Natriumsulfat, Eindampfen und chromatographischer Reinigung des Rückstandes mit Methylenchlorid erhält man 99,5 g (49,2 % d. Th.) 2-(2-Chlor-2-fluorethylthio)-phenyl-sulfonamid mit einem Schmelzpunkt von +88 °C bis +89 °C.

Beispiel H9: Herstellung von N-(2-(2-chlor-2-fluorethylthio)-phenyl-sulfonyl)-N'-(4-methoxy-6-methyl-1,3,5-triazin-2-yl)-harnstoff (Verbindung Nr. 1.001):

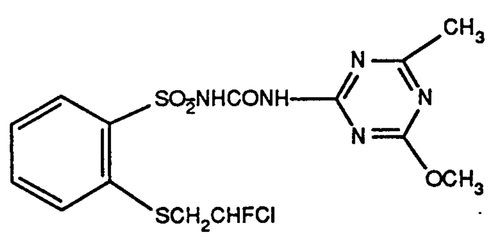

(1.001)

Zu einer Mischung von 1,9 g 2-(2-Chlor-2-fluorethylthio)-phenylsulfonamid und 1,85 g N-(-4-Methyl-6-methoxy-1,3,5-triazin-2-yl)-phenylcarbamat in 40 ml absolutem Dioxan gibt man bei einer Temperatur von +20 bis +25 °C eine Lösung aus 1,07 g 1,8-Diazabicyclo[5.4.0]undec-7-en in 10 ml absolutem Dioxan tropfenweise hinzu. Anschließend wird die Reaktionsmischung 6 Stunden gerührt und dann in Wasser gegeben. Nach Ansäuern mit 10%-iger Salzsäure, Extrahieren mit Ethylacetat, Waschen mit Wasser und Trocknen über Natriumsulfat wird die Reaktionsmischung filtriert. Nach Eindampfen des Filtrates und Umkristallisieren des Rückstandes aus Ethylacetat erhält man 2,4 g N-(2-(2-chlor-2-fluorethylthio)-phenylsulfonyl)-N'-(4-methoxy-6-methyl-1,3,5-triazin-2-yl)-harnstoff (Verbindung Nr. 1.001) mit einem Schmelzpunkt von +174 bis +176 °C.

Beispiel H10: Herstellung von N-(2-(2,2-difluorethylthio)-phenyl-sulfonyl)-N'-(4-methoxy-6-methyl-1,3,5-triazin-2-yl)-harnstoff (Verbindung Nr. 1.008):

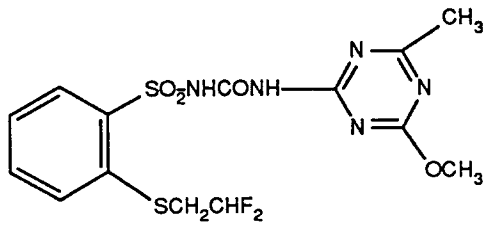

(1.008)

Zu einer Mischung von 1,77 g 2-(2,2-Difluorethylthio)-phenylsulfonamid und 1,82 g N-(4-Methyl-6-methoxy-1,3,5-triazin-2-yl)-phenylcarbamat in 40 ml absolutem Dioxan gibt man bei einer Temperatur von +20 bis +25°C eine Lösung aus 1,07 g 1,8-Diazabicyclo[5.4.0]undec-7-en in 10 ml absolutem Dioxan tropfenweise hinzu. Anschließend wird die Reaktionsmischung 6 Stunden gerührt und dann in Wasser gegeben. Nach Ansäuern mit 10%-iger Salzsäure, Extrahieren mit Ethylacetat, Waschen mit Wasser und Trocknen über Natriumsulfat wird die Reaktionsmischung filtriert. Nach Eindampfen des Filtrates und Umkristallisieren des Rückstandes aus Ethylacetat erhält man 2,3 g N-(2-(2,2-difluor-ethylthio)-phenyl-sulfonyl)-N'-(4-methoxy-6-methyl-1,3,5-triazin-2-yl)-harnstoff (Verbindung Nr. 1.001) mit einem Schmelzpunkt von +189 bis +190 °C.

In analoger Weise werden die in der angeschlossenen Tabelle aufgelisteten Verbindungen der Formel I hergestellt.

## Tabelle 1: Verbindungen der Formel I:

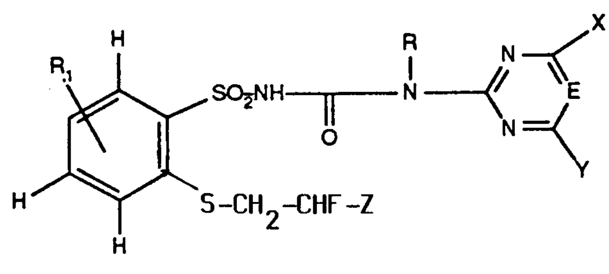

(I)

Tabelle 1:

| Verb. Nr. | Z | R | $R_1$ | Y | X | E | Smp. [°C] |
|---|---|---|---|---|---|---|---|
| 1.001 | Cl | H | H | $CH_3$ | $OCH_3$ | N | 174-176 |
| 1.002 | Cl | $CH_3$ | H | $CH_3$ | $OCH_3$ | N | |
| 1.003 | Cl | H | H | $CH_3$ | $OCH_3$ | CH | 159-161 |
| 1.004 | Cl | H | H | 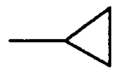 | $OC_2H_5$ | N | |
| 1.005 | Cl | H | H | 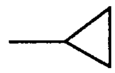 | $OCH_3$ | N | |
| 1.006 | Cl | H | H | $C_2H_5$ | $OCH_3$ | N | |
| 1.007 | Cl | H | H | $CH_3$ | $OC_2H_5$ | N | |
| 1.008 | F | H | H | $CH_3$ | $OCH_3$ | N | 189-190 |
| 1.009 | F | $CH_3$ | H | $CH_3$ | $OCH_3$ | N | |
| 1.010 | F | H | H | $CH_3$ | $OCH_3$ | CH | 170-174 |
| 1.011 | F | H | H | $CH_3$ | $OC_2H_5$ | N | |
| 1.012 | F | H | H | 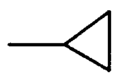 | $OC_2H_5$ | N | |
| 1.013 | F | H | H | 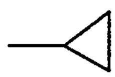 | $CH_3$ | CH | |
| 1.014 | F | H | H | 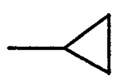 | $OCH_3$ | N | |
| 1.015 | F | H | H | $C_2H_5$ | $OCH_3$ | N | |
| 1.016 | F | H | H | $NHCH_3$ | $OC_2H_5$ | N | |
| 1.017 | F | H | H | $N(CH_3)_2$ | $OCH_3$ | N | |
| 1.018 | F | H | H | $N(CH_3)_2$ | $OCH_2CF_3$ | N | |
| 1.019 | F | H | H | Cl | $OCH_3$ | N | |
| 1.020 | F | H | H | $OCHF_2$ | $OCHF_2$ | CH | |
| 1.021 | F | H | H | $OCH_3$ | $OCH_3$ | N | 162-167 |
| 1.022 | F | H | H | $OCH_3$ | $OCH_3$ | CH | 179-181 |
| 1.023 | F | H | H | Cl | $OCH_3$ | CH | 154-160 |
| 1.024 | F | H | H | $OCHF_2$ | $OCH_3$ | CH | 170-172 |

Biologische Beispiele

Zur Überprüfung der herbiziden Aktivität der Verbindungen aus der vorliegenden Anmeldung gegenüber Verbindungen aus dem Stand der Technik (EP-A-0 044 808) wurden die folgenden Beispiele B1 und B2

durchgeführt:

Beispiel B1: Pre-emergente Herbizid-Wirkung

Im Gewächshaus wird unmittelbar nach der Einsaat der Versuchspflanzen (Amarantus, Chenopodium, Sinapis, Stellaria, Chrysanthemum, Galium und Veronica) in Saatschalen die Erdoberfläche mit einer wäßrigen Spritzbrühe entsprechend einer Aufwandmenge von 30 g bzw. 8 g Wirksubstanz/Hektar behandelt. Die Saatschalen werden im Gewächshaus bei 24 - 26 ° C und 45 - 60 % relativer Luftfeuchtigkeit gehalten.

Nach 3 Wochen wird die Herbizidwirkung mit einem neunstufigen Boniturschema im Vergleich in einer unbehandelten Kontrollgruppe bewertet:

1: Pflanzen nicht gekeimt oder total abgestorben
2-3: sehr starke Wirkung
4-6: mittlere Wirkung
7-8: geringe Wirkung
9: keine Wirkung (wie unbehandelte Kontrolle)

Die geprüften Wirkstoffe sind:

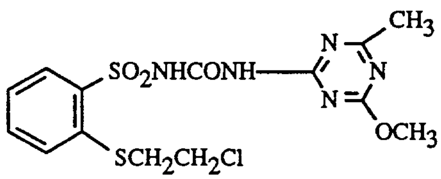

Verbindung A: (Nr. 391 aus EP-A-0 044 808) gegen:

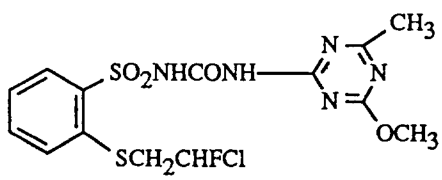

Nr. 1.001 aus der vorliegenden Anmeldung, sowie

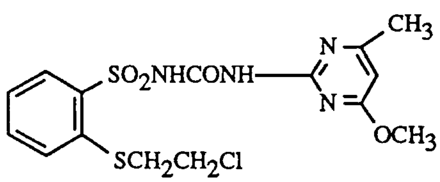

Verbindung B (Nr. 399 aus EP-A-0 044 808) gegen:

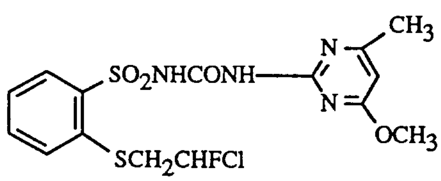

Nr. 1.003 aus der vorliegenden Anmeldung.

Die Resultate sind in den Tabellen T1 und T2 aufgeführt:

Tabelle T1

| preemergente Herbizidwirkung bei 30g AS/ha: | | | | |
|---|---|---|---|---|
| Testpflanze | Verb. A | Verb. 1.001 | Verb. B | Verb. 1.003 |
| Amarantus | 5 | 2 | 5 | 3 |
| Chenopodium | 9 | 2 | 5 | 2 |
| Sinapis | 8 | 2 | 8 | 2 |
| Stellaria | 9 | 2 | 9 | 2 |
| Chrysanth. | 9 | 2 | 5 | 2 |
| Galium | 9 | 3 | 9 | 2 |

Tabelle T2

| preemergente Herbizidwirkung bei 8g AS/ha: | | |
|---|---|---|
| Testpflanze | Verb. B | Verb. 1.003 |
| Amarantus | 9 | 7 |
| Sinapis | 9 | 3 |
| Stellaria | 9 | 2 |
| Chrysanth. | 9 | 2 |
| Galium | 9 | 3 |
| Veronica | 5 | 2 |

In diesem Versuch zeigten sich die Verbindungen der vorliegenden Anmeldung gegenüber den Verbindungen aus dem Stand der Technik in ihrer herbiziden Wirkung deutlich überlegen.

Beispiel B2: Post-emergente Herbizid-Wirkung (Kontaktherbizid)

Eine Anzahl Unkräuter ((Amarantus, Chenopodium, Sinapis, Stellaria, Chrysanthemum, Galium, Viola tricolor und Veronica) wurden nach dem Auflaufen (im 4- bis 6-Blattstadium) mit einer wäßrigen Wirkstoffdispersion in einer Dosierung von 8 bzw. 30 g Wirksubstanz pro Hektar auf die Pflanzen gespritzt und diese bei 24°-26°C und 45-60 % relativer Luftfeuchtigkeit gehalten. 15 Tage nach der Behandlung wurde der Versuch mit einem neunstufigen Boniturschema im Vergleich in einer unbehandelten Kontrollgruppe bewertet:

1: Pflanzen nicht gekeimt oder total abgestorben
2-3: sehr starke Wirkung
4-6: mittlere Wirkung
7-8: geringe Wirkung
9: keine Wirkung (wie unbehandelte Kontrolle)

Die Resultate sind in den Tabellen T3 und T4 aufgeführt:

Tabelle T3

| postemergente Herbizidwirkung bei 8 g AS/ha: | | |
|---|---|---|
| Testpflanze | Verb. A | Verb. 1.001 |
| Amarantus | 9 | 3 |
| Chenopodium | 9 | 4 |
| Sinapis | 9 | 3 |
| Stellaria | 9 | 3 |
| Chrysanth. | 9 | 5 |
| Galium | 9 | 8 |
| Viola t. | 9 | 7 |
| Veronica | 9 | 7 |

Tabelle T4

| postemergente Herbizidwirkung bei 30 g AS/ha: | | | | |
|---|---|---|---|---|
| Testpflanze | Verb. A | Verb. 1.001 | Verb. B | Verb. 1.003 |
| Amarantus | 7 | 2 | - | - |
| Chenopodium | 9 | 3 | 8 | 6 |
| Sinapis | 3 | 2 | 4 | 2 |
| Stellaria | 3 | 2 | - | - |
| Chrysanth. | 4 | 3 | - | - |
| Galium | 6 | 3 | 7 | 6 |
| Viola t. | 9 | 4 | 4 | 7 |
| Veronica | 9 | 7 | - | - |

Auch bei der postemergenten Applikation zeigten die Verbindungen der Formel I gemäß vorliegender Anmeldung eine bessere herbizide Wirkung als die Vergleichssubstanzen aus dem Stand der Technik.

Beispiel B3: Herbizidwirkung für Wasserreis (paddy) Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefäße. Die verwendete Dosis entspricht einer Wirkstoffmenge von 8-500 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Formel I schädigen dabei die Unkräuter.

Formulierungsbeispiele für Wirkstoffe der Formel I
(% = Gewichtsprozent)

1. Spritzpulver

| | a) | b) | c) |
|---|---|---|---|
| Wirkstoff der Formel I | 20 % | 50 % | 0,5 % |
| Na-Ligninsulfonat | 5 % | 5 % | 5 |
| Na-Laurylsulfat | 3 % | - | - % |
| Na-Diisobutylnaphthalinsulfonat | - | 6 % | 6 % |
| Octylphenolpolyethylenglykolether (7-8 Mol AeO) | - | 2% | 2 |
| Hochdisperse Kieselsäure | 5 % | 27 % | 27 % |
| Kaolin | 67 % | - % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrate

| | a) | b) |
|---|---|---|
| Wirkstoff der Formel I | 10% | 1 % |
| Ca-Dodecylbenzolsulfonat | 3 % | 3 % |
| Octylphenolpolyethylenglykolether (4-5 Mol AeO) | 3 % | 3 % |
| Ricinusöl-polyethylenglykolether (36 Mol AeO) | 4% | 4 |
| Cyclohexanon | 30 | 10 % |
| Xylolgemisch | 50 % | 79 % |

Aus solchen Konzentraten können durch Verdünnen mit Wasser Emulsionen jeder gewünschten Konzentration hergestellt werden.

3. Stäubemittel

| | a) | b) |
|---|---|---|
| Wirkstoff der Formel I | 0,1 % | 1 % |
| Talkum | 99,9 % | - |
| Kaolin | - | 99 % |

Durch inniges Vermischen der Trägerstoffe mit dem Wirkstoff erhält man gebrauchsfertige Stäubemittel.

4. Extruder-Granulat

| | a) | b) |
|---|---|---|
| Wirkstoff der Formel I | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschließend im Luftstrom getrocknet.

5. Umhüllungs-Granulat

| | |
|---|---|
| Wirkstoff der Formel I | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

6. Suspensions-Konzentrat

| | a) | b) |
|---|---|---|
| Wirkstoff der Formel I | 5 % | 40 % |
| Ethylenglykol | 10 % | 10 % |
| Nonylphenolpolyethylenglykolether (15 Mol AeO) | 1 % | 6 % |
| Na-Ligninsulfonat | 5 % | 10 % |
| Carboxymethylcellulose | 1 % | 1 % |
| 37 %ige wäßrige Formaldehyd-Lösung | 0,2 % | 0,2 % |
| Silikonöl in Form einer 75%-igen wäßrigen Emulsion | 0,8 % | 0,8 % |
| Wasser | 77 % | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

7. Salzlösung

| | |
|---|---|
| Wirkstoff der Formel I | 5 % |
| Isopropylamin | 1 % |
| Octylphenolpolyethylenglycolether (78 Mol AeO) | 3 % |
| Wasser | 91% |

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

## Patentansprüche

**1.** N-Phenylsulfonyl-N'-pyrimidinyl- und -triazinylharnstoffe der Formel I

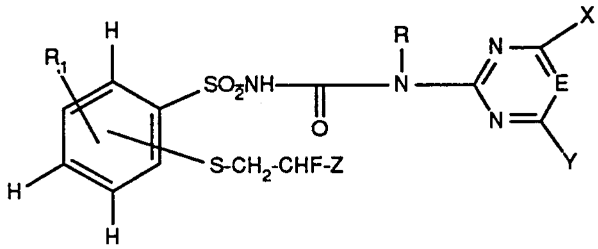

(I)

worin

R Wasserstoff oder $C_1$-$C_4$-Alkyl;

$R_1$ Wasserstoff, Halogen, $C_1$-$C_5$-Alkyl, $C_2$-$C_5$-Alkenyl, $C_1$-$C_4$-Halogenalkyl, COO-$R_2$ oder A-$R_3$;

$R_2$ $C_1$-$C_5$-Alkyl;

$R_3$ $C_1$-$C_5$-Alkyl, $C_2$-$C_4$-Alkoxyalkyl oder $C_1$-$C_5$-Halogenalkyl;

X $C_1$-$C_3$-Alkyl, durch Halogen ein- bis dreifach substituiertes $C_1$-$C_3$-Alkyl; $C_1$-$C_3$-Alkoxy oder durch Halogen ein- bis dreifach substituiertes $C_1$-$C_3$-Alkoxy;
A Sauerstoff, Schwefel, SO oder $SO_2$;
Y Halogen, $C_1$-$C_3$-Alkyl, durch Halogen ein- bis dreifach substituiertes $C_1$-$C_3$-Alkyl; $C_1$-$C_3$-Alkoxy, durch Halogen ein- bis dreifach substituiertes $C_1$-$C_3$-Alkoxy; Cyclopropyl, Methylamino oder Dimethylamino;
E Stickstoff oder die Methingruppe; und
Z Fluor oder Chlor bedeuten;
sowie die Salze dieser Verbindungen.

**2.** Verbindungen gemäß Anspruch 1, dadurch gekennzeichnet, daß $R_1$ am Phenylring in 5-Stellung und die Gruppe -S-$CH_2$-CHF-Z in 2-Stellung steht.

**3.** Verbindungen gemaß Anspruch 1, dadurch gekennzeichnet, daß R Wasserstoff bedeutet.

**4.** Verbindungen gemäß Anspruch 1, dadurch gekennzeichnet, daß $R_1$ Wasserstoff bedeutet.

**5.** Verbindungen der Formel I gemaß Anspruch 1, dadurch gekennzeichnet, daß E für Stickstoff steht.

**6.** Verbindungen gemäß Anspruch 1, dadurch gekennzeichnet, daß X für $C_1$-$C_3$-Alkyl, $C_1$-$C_3$-Alkoxy oder durch Halogen ein- bis dreifach substituiertes $C_1$-$C_3$-Alkoxy; und Y für Chlor, $C_1$-$C_3$-Alkyl, $C_1$-$C_3$-Alkoxy, Cyclopropyl oder durch Halogen ein- bis dreifach substituiertes $C_1$-$C_3$-Alkoxy steht.

**7.** Verbindungen der Formel I gemäß Anspruch 1, dadurch gekennzeichnet, daß X für Methyl, Methoxy, Ethoxy oder Difluormethoxy; und Y für Methyl, Methoxy, Ethoxy, Difluormethoxy oder Chlor steht.

**8.** Verbindungen der Formel I gemäß Anspruch 1, dadurch gekennzeichnet, daß X für Methoxy oder Ethoxy; und Y für Methyl oder Methoxy steht.

**9.** Verbindungen gemäß Anspruch 4, dadurch gekennzeichnet, daß X für $C_1$-$C_3$-Alkyl, $C_1$-$C_3$-Alkoxy oder durch Halogen ein- bis dreifach substituiertes $C_1$-$C_3$-Alkoxy; und Y für Chlor, $C_1$-$C_3$-Alkyl, $C_1$-$C_3$-Alkoxy, Cyclopropyl oder durch Halogen ein- bis dreifach substituiertes $C_1$-$C_3$-Alkoxy steht.

**10.** Verbindungen der Formel I gemäß Anspruch 9, dadurch gekennzeichnet, daß X für Methyl, Methoxy, Ethoxy oder Difluormethoxy; und Y für Methyl, Methoxy, Ethoxy, Difluormethoxy oder Chlor steht.

**11.** Verbindungen der Formel I gemäß Anspruch 9, dadurch gekennzeichnet, daß X für Methoxy oder Ethoxy; und Y für Methyl oder Methoxy steht.

**12.** Verbindungen der Formel I gemäß Anspruch 9, dadurch gekennzeichnet, daß die Gruppe -S-$CH_2$-CHF-Z am Phenylring in 2-Stellung steht.

**13.** N-(2-(2-chlor-2-fluorethylthio)-phenyl-sulfonyl)-N'-(4-methoxy-6-methyl-1,3,5-triazin-2-yl)-harnstoff gemäß Anspruch 1.

**14.** N-(2-(2,2-difluor-ethylthio)-phenyl-sulfonyl)-N'-(4-methoxy-6-methyl-1,3,5-triazin-2-yl)-harnstoff gemaß Anspruch 1.

**15.** Verfahren zur Herstellung der Verbindungen der Formel I gemäß Anspruch 1, dadurch gekennzeichnet, daß man

a) ein Phenylsulfonamid der Formel XIV

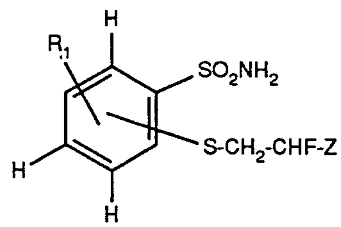

(XIV)

worin Z die unter Formel I angegebene Bedeutung hat, mit einem N-Pyrimidinyl- oder N-Triazinyl-carbamat der Formel XI

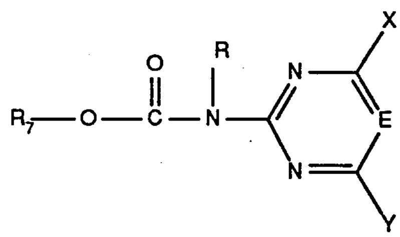

(XI)

worin X, Y, R und E die unter Formel I in Anspruch 1 angegebene Bedeutung haben und $R_7$ $C_1$-$C_4$-Alkyl, oder Phenyl, welches durch $C_1$-$C_4$-Alkyl oder Halogen substituiert sein kann, bedeutet; in Gegenwart einer Base umsetzt, oder

b) ein Phenylsulfonamid der Formel XII

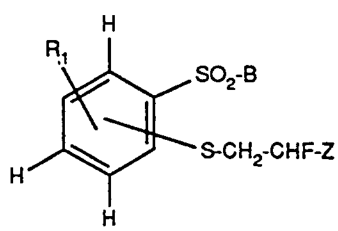

(XII)

worin $R_1$ und Z die in Formel I im Anspruch 1 angegebene Bedeutung haben, und B

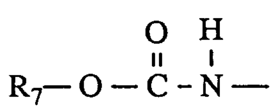

oder O=C=H- bedeutet, in Gegenwart einer Base mit einem 2-Aminopyrimidin- oder -triazin der Formel XIII

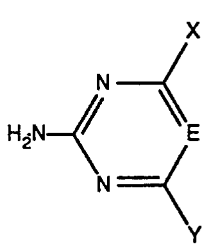

(XIII)

worin E, X und Y die unter Formel I in Anspruch 1 angegebene Bedeutung haben, umsetzt.

16. Ein herbizides und den Pflanzenwuchs hemmendes Mittel, gekennzeichnet durch ein Gehalt an einem oder mehreren Sulfonylharnstoffen der Formel I, gemäß Anspruch 1.

17. Mittel gemäß Anspruch 16, dadurch gekennzeichnet, daß es zwischen 0,1 % und 95 % Wirkstoff der Formel I gemaß Anspruch 1 enthält.

18. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

19. Verfahren gemäß Anspruch 18, dadurch gekennzeichnet, daß man eine Wirkstoffmenge zwischen 0,001 und 2 kg pro Hektar appliziert.

20. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

21. Verfahren gemäß Anspruch 18, zur selektiven pre- oder post-emergenten Bekämpfung von Unkräutern in Nutzpflanzenkulturen.

**Claims**

1. An N-phenylsulfonyl-N'-pyrimidinyl- or -triazinyl-urea of formula I

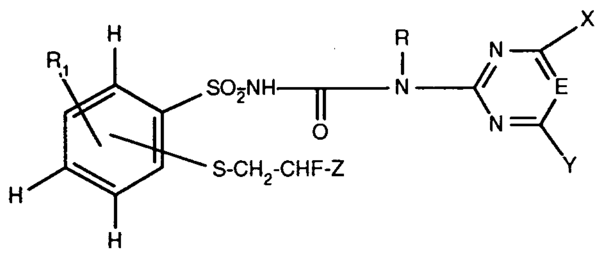

(I)

wherein
R is hydrogen or $C_1$-$C_4$alkyl;
$R_1$ is hydrogen, halogen, $C_1$-$C_5$alkyl, $C_2$-$C_5$alkenyl, $C_1$-$C_4$haloalkyl, COO-$R_2$ or A-$R_3$;
$R_2$ is $C_1$-$C_5$alkyl;
$R_3$ is $C_1$-$C_5$alkyl, $C_2$-$C_4$alkoxyalkyl or $C_1$-$C_5$haloalkyl;
X is $C_1$-$C_3$alkyl, $C_1$-$C_3$alkyl mono- to tri-substituted by halogen; $C_1$-$C_3$alkoxy or $C_1$-$C_3$alkoxy mono- to tri-substituted by halogen;
A is oxygen, sulfur, SO or $SO_2$;
Y is halogen, $C_1$-$C_3$alkyl, $C_1$-$C_3$alkyl mono- to tri-substituted by halogen; $C_1$-$C_3$alkoxy, $C_1$-$C_3$alkoxy mono- to tri-substituted by halogen; cyclopropyl, methylamino or dimethylamino;
E is nitrogen or the methine group; and
Z is fluorine or chlorine;
or a salt of that compound.

2. A compound according to claim 1, wherein
$R_1$ at the phenyl ring is in the 5-position and the group -S-$CH_2$-CHF-Z is in the 2-position.

3. A compound according to claim 1, wherein
R is hydrogen.

4. A compound according to claim 1, wherein
$R_1$ is hydrogen.

**5.** A compound of formula I according to claim 1, wherein E is nitrogen.

**6.** A compound according to claim 1, wherein X is $C_1$-$C_3$alkyl, $C_1$-$C_3$alkoxy or $C_1$-$C_3$alkoxy mono- to tri-substituted by halogen; and Y is chlorine, $C_1$-$C_3$alkyl, $C_1$-$C_3$alkoxy, cyclopropyl, or $C_1$-$C_3$alkoxy mono- to tri-substituted by halogen.

**7.** A compound of formula I according to claim 1, wherein X is methyl, methoxy, ethoxy or difluoromethoxy; and Y is methyl, methoxy, ethoxy, difluoromethoxy or chlorine.

**8.** A compound of formula I according to claim 1, wherein X is methoxy or ethoxy; and Y is methyl or methoxy.

**9.** A compound according to claim 4, wherein X is $C_1$-$C_3$alkyl, $C_1$-$C_3$alkoxy or $C_1$-$C_3$alkoxy mono- to tri-substituted by halogen; and Y is chlorine, $C_1$-$C_3$alkyl, $C_1$-$C_3$alkoxy, cyclopropyl, or $C_1$-$C_3$alkoxy mono- to tri-substituted by halogen.

**10.** A compound of formula I according to claim 9, wherein X is methyl, methoxy, ethoxy or difluoromethoxy; and Y is methyl, methoxy, ethoxy, difluoromethoxy or chlorine.

**11.** A compound of formula I according to claim 9, wherein X is methoxy or ethoxy; and Y is methyl or methoxy.

**12.** A compound of formula I according to claim 9, wherein the group $-S-CH_2-CHF-Z$ on the phenyl ring is in the 2-position.

**13.** N-(2-(2-chloro-2-fluoroethylthio)-phenylsulfonyl)-N'-(4-methoxy-6-methyl-1,3,5-triazin-2-yl)-urea according to claim 1.

**14.** N-(2-(2,2-difluoroethylthio)-phenylsulfonyl)-N'-(4-methoxy-6-methyl-1,3,5-triazin-2-yl)-urea according to claim 1.

**15.** A process for the preparation of a compound of formula I according to claim 1, which comprises

a) reacting a phenylsulfonamide of formula XIV

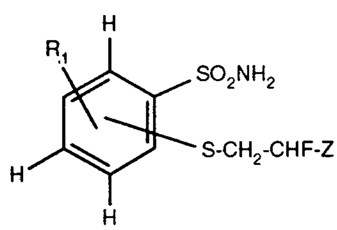

(XIV)

wherein Z is as defined under formula I, in the presence of a base, with an N-pyrimidinyl or N-triazinyl carbamate of formula XI

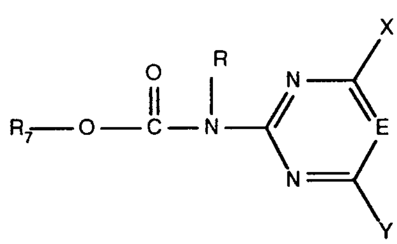

(XI)

wherein X, Y, R and E are as defined under formula I in claim 1 and $R_7$ is $C_1$-$C_4$alkyl, or phenyl which may be substituted by $C_1$-$C_4$alkyl or by halogen; or

b) reacting a phenylsulfonamide of formula XII

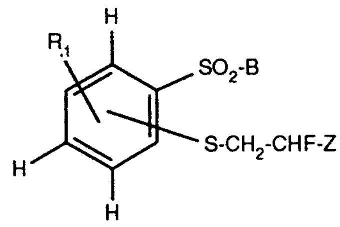

(XII)

wherein $R_1$ and Z are as defined in formula I in claim 1, and B is

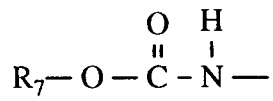

or O = C = N-, in the presence of a base, with a 2-amino-pyrimidine or -triazine of formula XIII

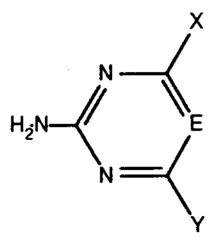

(XIII)

wherein E, X and Y are as defined under formula I in claim 1.

**16.** A herbicidal and plant-growth-inhibiting composition comprising one or more sulfonylureas of formula I, according to claim 1.

**17.** A composition according to claim 16 comprising a compound of formula I according to claim 1 in an amount of from 0.1 % to 95 %.

**18.** A method of controlling undesirable plant growth, which comprises applying to the plants or to the locus thereof an effective amount of a compound of formula I, according to claim 1, or of a composition comprising that compound.

**19.** A method according to claim 18, which comprises applying the active ingredient in an amount of from 0.001 to 2 kg per hectare.

**20.** A method of inhibiting plant growth, which comprises applying to the plants or to the locus thereof an effective amount of a compound of formula I, according to claim 1, or of a composition comprising that compound.

**21.** A method according to claim 18 for the selective pre- or post-emergence control of weeds in crops of useful plants.

## Revendications

**1.** N-phénylsulfonyl-N'-pyrimidinyl- et -triazinylurées de formule I

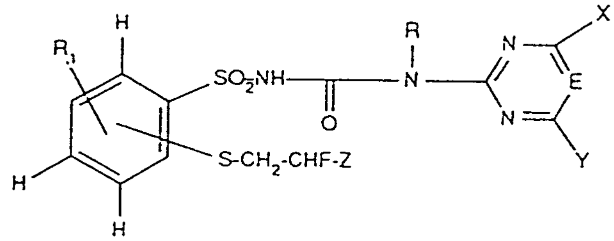

(I)

dans laquelle
R représente l'hydrogène ou un groupe alkyle en C1-C4;
$R_1$ représente l'hydrogène, un halogène, un groupe alkyle en C1-C5, alcényle en C2-C5, halogénoalkyle en C1-C4, COO-$R_2$ ou A-$R_3$;
$R_2$ représente un groupe alkyle en C1-C5;
$R_3$ représente un groupe alkyle en C1-C5, alcoxyalkyle en C2-C4 ou halogénoalkyle en C1-C5;
X représente un groupe alkyle en C1-C3, un groupe alkyle en C1-C3 mono- à tri-substitué par des halogènes; un groupe alcoxy en C1-C3 ou un groupe alcoxy en C1-C3 mono- à tri-substitué par des halogènes;
A représente l'oxygène, le soufre, SO ou $SO_2$;
Y représente un halogène, un groupe alkyle en C1-C3, un groupe alkyle en C1-C3 mono- à tri-substitué par des halogènes; un groupe alcoxy en C1-C3, un groupe alcoxy en C1-C3 mono- à tri-substitué par des halogènes; un groupe cyclopropyle, méthylamino ou diméthylamino ;
E représente l'azote ou le groupe méthine; et
Z représente le fluor ou le chlore; et les sels de ces composés.

**2.** Composés selon revendication 1, caractérisés en ce que $R_1$, sur le noyau phényle, est en position 5, et le groupe -S-$CH_2$-CHF-Z est en position 2.

**3.** Composés selon revendication 1, caractérisés en ce que R représente l'hydrogène.

**4.** Composés selon revendication 1, caractérisés en ce que $R_1$ représente l'hydrogène.

**5.** Composés de formule I selon revendication 1, caractérisés en ce que E représente l'azote.

**6.** Composés selon revendication 1, caractérisés en ce que
X représente un groupe alkyle en C1-C3, un groupe alcoxy en C1-C3 ou un groupe alcoxy en C1-C3 mono- à tri-substitué par des halogènes ; et
Y représente le chlore, un groupe alkyle en C1-C3, alcoxy en C1-C3, cyclopropyle ou alcoxy en C1-C3 mono- à tri-substitué par des halogènes.

**7.** Composés de formule I selon revendication 1, caractérisés en ce que
X représente un groupe méthyle, méthoxy, éthoxy ou difluorométhoxy ; et
Y représente un groupe méthyle, méthoxy, éthoxy, difluorométhoxy ou le chlore.

**8.** Composés de formule I selon revendication 1, caractérisés en ce que X représente un groupe méthoxy ou éthoxy et Y un groupe méthyle ou méthoxy.

**9.** Composés selon revendication 4, caractérisés en ce que
X représente un groupe alkyle en C1-C3, alcoxy en C1-C3 ou alcoxy en C1-C3 mono- à tri-substitué par des halogènes ; et
Y représente le chlore, un groupe alkyle en C1-C3, alcoxy en C1-C3, cyclopropyle ou alcoxy en C1-C3 mono- à tri-substitué par des halogènes.

**10.** Composés de formule I selon revendication 9, caractérisés en ce que X représente un groupe méthyle, méthoxy, éthoxy ou difluorométhoxy ; et Y représente un groupe méthyle, méthoxy, éthoxy, difluorométhoxy ou le chlore.

**11.** Composés de formule I selon revendication 9, caractérisés en ce que X représente un groupe méthoxy ou éthoxy et Y un groupe méthyle ou méthoxy.

**12.** Composés de formule I selon revendication 9, caractérisés en ce que le groupe $-S-CH_2-CHF-Z$ est en position 2 sur le noyau phényle.

**13.** La N-(2-(2-chloro-2-fluoréthylthio)phénylsulfonyl)-N'-(4-méthoxy-6-méthyl-1,3,5-triazine-2-yl)urée selon revendication 1.

**14.** La N-(2-(2,2-difluoréthylthio)phénylsulfonyl)-N'-(4-méthoxy-6-méthyl-1,3,5-triazine-2-yl)urée selon revendication 1.

**15.** Procédé de préparation des composés de formule I selon revendication 1, caractérisé en ce que
a) on fait réagir un phénylsulfonamide de formule XIV

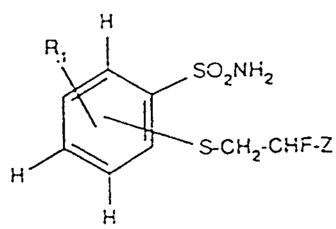

(XIV)

dans laquelle Z a les significations indiquées en référence à la formule I, avec un N-pyrimidinyl- ou N-triazinylcarbamate de formule XI

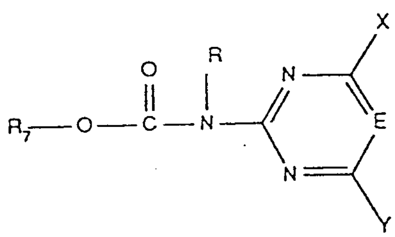

(XI)

dans laquelle X, Y, R et E ont les significations indiquées en référence à la formule I dans la revendication 1, et $R_7$ représente un groupe alkyle en C1-C4 ou un groupe phényle qui peut être substitué par un groupe alkyle en C1-C4 ou par un halogène, en présence d'une base, ou bien
b) on fait réagir un phénylsulfonamide de formule XII

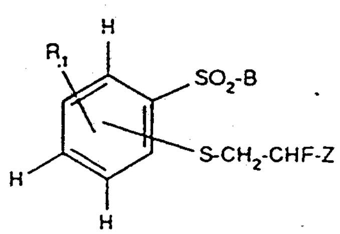

(XII)

dans laquelle $R_1$ et Z ont les significations indiquées en référence à la formule I dans la revendication 1, et B représente

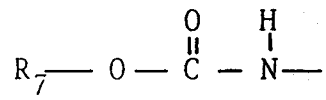 ou $O=C=N$

en présence d'une base, avec une 2-aminopyrimidine ou - triazine de formule XIII

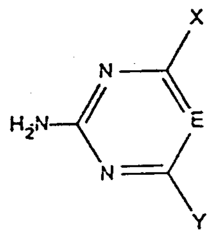

(XIII)

dans laquelle E, X et Y ont les significations indiquées en référence à la formule I dans la revendication 1.

**16.** Un produit herbicide et inhibiteur de la croissance des végétaux, caractérisé en ce qu'il contient une ou plusieurs sulfonylurées de formule I selon revendication 1.

**17.** Produit selon revendication 16, caractérisé en ce qu'il contient de 0,1% à 95% d'une substance active de formule I selon revendication 1.

**18.** Procédé pour combattre les croissances de végétaux indésirables, caractérisé en ce que l'on applique une substance active de formule I selon revendication 1 ou un produit contenant une telle substance active, en quantité efficace, sur les végétaux ou leur habitat.

**19.** Procédé selon revendication 18, caractérisé en ce que l'on applique une quantité de substance active de 0,001 à 2 kg par ha.

**20.** Procédé pour inhiber la croissance des végétaux, caractérisé en ce que l'on applique une substance active de formule I selon revendication 1 ou un produit contenant une telle substance active, en quantité efficace, sur les végétaux ou leur habitat.

**21.** Procédé selon revendication 18, pour la lutte sélective en pré-levée ou en post-levée contre les végétaux adventices dans les cultures de végétaux utiles.